# EUROPÄISCHE PATENTSCHRIFT

(11) **EP 3 182 951 B1**
(45) Veröffentlichungstag und Bekanntmachung des Hinweises auf die Patenterteilung: **24.06.2026**
(21) Anmeldenummer: 15735858.1
(22) Anmeldetag: 09.07.2015
(51) Int. Cl.: A61G 12/00, A61G 13/10, A61B 90/50

(54) **KRAFT- ODER BEWEGUNGSREAKTIVE STATIVVORRICHTUNG, STEUERUNGSEINRICHTUNG SOWIE VERFAHREN ZUM POSITIONIEREN DER STATIVVORRICHTUNG**
FORCE OR MOVEMENT REACTIVE STAND DEVICE, CONTROL DEVICE AND METHOD FOR POSITIONING THE STAND DEVICE
DISPOSITIF À PIED RÉACTIF AU MOUVEMENT ET À LA FORCE, DISPOSITIF DE COMMANDE ET PROCÉDÉ DE POSITIONNEMENT DU DISPOSITIF À PIED

(30) Priorität: 18.08.2014 EP 14002864
(43) Veröffentlichungstag der Anmeldung: 28.06.2017
(73) Patentinhaber: Ondal Medical Systems GmbH, 36088 Hünfeld (DE)
(72) Erfinder: PERPLIES, Stefan, 36088 Hünfeld (DE); VOLKENAND, Kai, 36088 Hünfeld (DE)
(74) Vertreter: Graf von Stosch, Andreas
(86) Internationale Anmeldenummer: PCT/EP2015/001398
(87) Internationale Veröffentlichungsnummer: WO 2016/026547

(56) Entgegenhaltungen:
- JP-A- 2009 291 363

## Beschreibung

Die vorliegende Erfindung betrifft eine Stativvorrichtung zur Anordnung in einem Operationssaal und zum örtlichen Verlagern einer medizintechnischen Einrichtung, umfassend eine Bremseinrichtung mit mindestens einer Bremse, die eingerichtet ist, einen Bewegungsfreiheitsgrad der medizintechnischen Einrichtung einzustellen. Die vorliegende Erfindung betrifft insbesondere eine Stativvorrichtung mit einzelnen Merkmalen des Anspruchs 1 sowie ein Verfahren zum Positionieren einer medizintechnischen Einrichtung mit einzelnen Merkmalen des entsprechenden unabhängigen Verfahrensanspruchs 12.

Stative, insbesondere Deckenstative wie z.B. Deckenversorgungseinheiten, Monitorträger, oder so genannte Federarme oder Zentralachsen, weisen meist einen oder mehrere in Bezug auf eine Vertikalposition starr angeordneten oder höhenverstellbaren Träger/Tragarm auf, mittels welchen eine daran befestigte medizintechnische Einrichtung bewegt und positioniert werden kann, z.B. in einem Operationssaal, insbesondere auch auf einer Intensivstation. An den Stativen sind häufig Versorgungseinheiten montiert, und/oder z.B. medizinisch-elektrische Endgeräte, die z.B. während einer Operation mit den benötigten Medien versorgt werden. Die Träger definieren dabei einen Aktionsradius der medizintechnischen Einrichtung, in welchem die medizintechnische Einrichtung positioniert werden kann. Die Träger können meist zumindest um mindestens eine drehbare Verbindung, insbesondere ein Drehgelenk verdreht werden. Wahlweise sind die Träger auch höhenverstellbar und/oder um eine zumindest annähernd horizontal ausgerichtete Achse in der Höhe verschwenkbar angeordnet. Die Höhenverstellbarkeit ist insbesondere bei Deckenstativen eine vorteilhafte Funktion.

Das Stativ bzw. die jeweilige medizintechnische Einrichtung muss dabei in Hinblick auf eine bestimmte Operation, einen bestimmten Patienten oder bestimmte Versorgungsleitungen in spezifischen Positionen fest angeordnet werden können. Deshalb weisen die Stative meist eine Bremseinrichtung oder irgendeine Blockierfunktion auf, welche sicherstellen kann, dass die medizintechnische Einrichtung in einer vorgegebenen Position verbleibt, selbst für den Fall dass Personen oder Gegenstände dagegen stoßen.

Andererseits soll das Stativ bzw. die jeweilige medizintechnische Einrichtung auch auf einfache Weise umpositioniert werden können, sei es während einer Operation oder zwischen zwei aufeinanderfolgenden Operationen oder bei unterschiedlichen Patienten oder Patienten-Liegen. Idealerweise soll das Stativ oder die medizintechnische Einrichtung intuitiv und ohne großen Kraftaufwand in eine bestimmte Position verlagert werden können, selbst wenn es eine Vielzahl von Tragarmen aufweist. Eine intuitive Bedienung/Positionierung der jeweiligen medizintechnischen Einrichtung ist dabei auch bei schlechten Lichtverhältnissen oder in Stresssituationen wünschenswert.

Da es sich bei den Stativen oder medizintechnischen Einrichtung um Vorrichtungen mit bedeutender Masse handeln kann, kann ein Verlagern oder Umpositionieren der medizintechnischen Einrichtung meist zumindest unterstützend auch durch motorische Antriebe erfolgen, insbesondere hinsichtlich einer Auf- und Abbewegung. Eine Drehbewegung einzelner Träger, sei es eine absolute Drehbewegung oder eine Drehbewegung relativ zu einem anderen Träger, soll dabei in vielen Fällen möglichst auch auf automatische bzw. motorische Weise abgebremst werden können. Hierdurch kann z.B. vermieden werden, dass ein Träger um mehr als einen vorgegebenen Winkel gegenüber einem anderen Träger verdreht wird. Das automatische Abbremsen hat auch den Vorteil, dass der einzelne Träger bzw. die medizintechnische Einrichtung nicht während der gesamten Bewegung manuell geführt werden muss. Ein motorisches oder automatisches Bremsen kann z.B. in einem Drehgelenk oder einem Lager erfolgen. Dabei kann die Bremswirkung einer z.B. friktionsbehafteten Bremse beispielsweise durch eine Schraube eingestellt oder nachjustiert werden.

Zum Umpositionieren der medizintechnischen Einrichtung muss von einem Bediener eine jeweilige Bremseinrichtung üblicherweise zunächst durch eine manuelle Betätigung eines Bedienknopfes oder Tasters gelöst oder freigegeben werden, insbesondere eines am Träger, an einem Bedienpanel oder einer Normschiene einer Versorgungskonsole angeordneten Bedienknopfes. Daraufhin kann dann die medizintechnische Einrichtung oder der Träger manuell und/oder motorisch verlagert werden. Mit anderen Worten erfolgt ein Umpositionieren in Reaktion auf eine Interaktion des Bedieners mit einem Schalter oder Taster. Der Bediener nimmt zunächst eine Betätigung an bestimmten Tasten vor, um einzelne Bremsen zu öffnen und eine Bewegung des jeweiligen Trägers zu ermöglichen.

Diese Art der Bedienung hat Nachteile. Speziell bei komplexeren Stativen mit einer Vielzahl von medizintechnischen Einrichtungen (z.B. auch Monitoren, Leuchten) kann es erforderlich sein, erst den entsprechenden Schalter zu suchen, was Zeit und Nerven rauben kann.

Die europäische Patentschrift EP 2 455 053 B1 beachtet ebenfalls dieses Problem und schlägt als Lösung vor, den Bediener durch eine Rückmeldung zu informieren, ob jeweils die gewünschte/richtige Bremse eines Stativs mittels des entsprechenden Tasters/Schalters betätigt wurde. Dabei wird vorgeschlagen, bei der Systemsteuerung in Reaktion auf eine Bedieneingabe eine Zeitverzögerung vorzusehen. Im Falle einer Fehlbedienung hat der Bediener dann noch Zeit, den Fehler zu korrigieren und den korrekten Schalter zu betätigen.

Aus der JP 2009/291363 ist eine Armstützvorrichtung bekannt, die einen Armtisch, einen Mehrfachgelenkarm mit einer Vielzahl von Gelenken zum beweglichen Tragen des Armtischs aufweist, eine Mehrzahl an Federn, um die Gelenke in ihren jeweiligen Stellungen zu halten, eine Mehrzahl von Pulverbremsen zum Steuern des Bremsmoments zum Abbremsen einer Bewegung der Gelenke, eine Mehrzahl von Encodern zum Erfassen der in jedem der Gelenke durchgeführten Operationen, ein 6-Achs-Drehmomentsensor zum Erfassen der Kraft und des Drehmoments, das auf den Armtisch ausgeübt wird, und ein Steuerteil zum Steuern der jeweiligen Bremsenergie der Pulverbremsen, basierend auf einem, von einem Fußschalter vorgegebenen Modus und den Detektionswerten des 6-Achs-Drehmomentsensors sowie der Encoder. Ein Lösen der Pulverbremsen ist dabei nur über eine Betätigung des Fußschalters möglich.

Es ist eine Aufgabe der vorliegenden Erfindung, eine Vorrichtung bereitzustellen, insbesondere eine Stativvorrichtung mit einer Bremseinrichtung, mittels welcher ein Bediener eine medizintechnische Einrichtung auf einfache oder intuitive Weise verlagern kann, sei es rein manuell oder motorisch unterstützt. Insbesondere besteht die Aufgabe auch darin, eine Stativvorrichtung bereitzustellen, bei welcher einzelne Träger oder Tragarme der Stativvorrichtung dank einer auf einfache Weise einstellbaren Bremseinrichtung auf besonders einfache oder flexible Weise im Operationssaal verlagert werden können, insbesondere ohne dass besonders viel Aufmerksamkeit des Bedieners dazu erforderlich ist. Die Aufgabe kann auch darin gesehen werden, ein Verfahren bereitzustellen, mittels welchem die Stativvorrichtung auf unkomplizierte oder intuitive Weise verlagert oder umpositioniert werden kann.

Diese Aufgabe wird gelöst durch eine Stativvorrichtung zur Anordnung im Operationssaal und zum örtlichen Verlagern einer an der Stativvorrichtung montierten medizintechnischen Einrichtung, umfassend (i) ein Tragsystem, umfassend mindestens einen bewegbar gelagerten Tragarm und eine Abstützeinrichtung; und (ii) eine Bremseinrichtung mit mindestens einer Bremse, die eingerichtet ist, einen Bewegungsfreiheitsgrad des mindestens einen Tragarms und/oder der medizintechnischen Einrichtung relativ zur Abstützeinrichtung einzustellen; wobei die Stativvorrichtung ferner umfasst:
eine mit der Bremseinrichtung in Verbindung stehende Steuerungseinrichtung und mindestens eine mit der Steuerungseinrichtung in Verbindung stehende Sensoreinrichtung eingerichtet zum Erfassen einer auf die Stativvorrichtung einwirkenden externen Kraft und/oder einer durch die externe Kraft hervorgerufenen Bewegung, und eingerichtet zum Ausgeben eines entsprechenden Messwerts an die Steuerungseinrichtung, wobei die Steuerungseinrichtung eingerichtet ist, die Bremseinrichtung in Abhängigkeit des Messwerts anzusteuern und den Bewegungsfreiheitsgrad einzustellen, und wobei die Steuerungseinrichtung (30) eingerichtet ist, in Abhängigkeit von einer zeitlichen Änderung, einem Betrag und/oder einer Richtung der externen Kraft und/oder einer Richtung und/oder einer Geschwindigkeit der Bewegung den Bewegungsfreiheitsgrad der Stativvorrichtung (1) einzustellen, wobei die Steuerungseinreichtung eingerichtet ist, die Bremseinrichtung in Anhängigkeit des Messwerts autonom und ohne Betätigung eines zusätzlichen Schalters ausschließlich aufgrund der auf die Stativvorrichtung einwirkenden externen Kraft zu lösen.

Wahlweise kann die durch die externe Kraft hervorgerufene Bewegung des mindestens einen Tragarms, insbesondere einer Bewegung relativ zur Abstützeinrichtung, erfasst werden.

Hierdurch kann das Stativ oder die medizintechnische Einrichtung intuitiv und ohne großen Bedienungs- oder Kraftaufwand in eine bestimmte Position verlagert werden, sei es durch OP-Personal oder durch einen Operateur, insbesondere auch auf einfache Weise während einer Operation (häufig wiederholtes Umpositionieren). Das Lösen der Bremsvorrichtung erfolgt also nur aufgrund der auf die Stativvorrichtung ausgeübten Kraft. Ein Taster oder Schalter zum Lösen irgendeiner Bremse muss dabei nicht betätigt werden. Bestimmte Kenntnisse über die Position von irgendwelchen Tastern oder Schaltern sind nicht erforderlich. Vielmehr kann jegliche Redundanz irgendwelcher Schalter oder Tasten/Taster vermieden werden. Ebenso wenig muss irgendeine Taste zum Feststellen der Bremse nach erfolgter Verlagerung bedient werden. Auch muss kein Schalter oder keine Taste kontinuierlich während einem Umpositionieren gedrückt gehalten werden. Ein Bediener muss sich gar nicht mehr mit der Frage auseinandersetzen, an welchen Stellen des Stativs welche Schalter angeordnet sind. Vielmehr reicht z.B. Druck auf einen seitlichen Teil eines Tragarms oder Betätigung eines ohnehin für den Bediener gut zugänglichen Bedientabletts, um das Stativ umzupositionieren. Auch kann z.B. eine Normschiene einer Versorgungskonsole als Angriffspunkt dienen, um eine externe Kraft zu erfassen.

Das Erfassen einer Bewegung kann dabei wahlweise das Erfassen einer translatorischen und/oder einer rotatorischen Bewegung umfassen. Die Sensoreinrichtung kann beispielsweise ein Inkrementalgeber sein oder einen Inkrementalgeber umfassen, sei es für einen Winkel und/oder für eine geradlinige Wegstrecke.

Der Bewegungsfreiheitsgrad kann auch dadurch eingestellt werden, dass eine Bremse gelockert wird, aber nicht komplett gelöst wird. Dies ist insbesondere in Hinblick auf Notlaufeigenschaften oder die Sicherheit und Flexibilität während einer Operation vorteilhaft. Insbesondere kann eine die medizintechnische Einrichtung nicht blockierende, sondern nur z.B. mit einem Bremsmoment von 70Nm haltende Bremse gelockert oder gelöst werden bzw. wieder in Hinblick auf ein spezifisches Bremsmoment festgestellt/geschlossen werden. Eine Bremseinrichtung, welche nicht blockiert, sondern nur ein im Notfall überwindbares Bremsmoment bereitstellt, liefert einen hohen praktischen Nutzen, insbesondere für einen behandelnden Arzt. Das Lockern einer Bremse kann dabei beispielsweise auch in Höhe eines vordefinierbaren Schwellwerts erfolgen, z.B. 30Nm, oder 50Nm, insbesondere derart, dass auch eine "freigegebene" oder lockere Bremseinrichtung noch ein Mindest-Bremsmoment z.B. im Bereich von 10Nm oder 20Nm bereitstellt.

Ein Lösen der Bremseinrichtung kann dabei auch ein Lockern der Bremseinrichtung umfassen, bei welchem noch ein Mindest-Bremsmoment bzw. eine Mindest-Bremskraft aufrechterhalten wird. Das Lösen kann dabei auch zeitbezogen erfolgen, insbesondere in Abhängigkeit eines vordefinierbaren Kraft- oder Momentenanstiegs. Hierdurch kann auch ausgewertet werden, ob eine externe Kraft beispielsweise nur durch versehentlichen Anstoßen der Stativvorrichtung hervorgerufen wurde.

Dabei kann die Stativvorrichtung kraftreaktiv und/oder bewegungsreaktiv sein, insbesondere bezüglich der auf die Stativvorrichtung ausgeübten externen Kraft oder dadurch hervorgerufenen Bewegung. Die Stativvorrichtung kann dabei auch positionsreaktiv sein, also in Abhängigkeit einer bestimmten Position einen bestimmten Bewegungsfreiheitsgrad aufweisen. Entsprechend ist die Steuerungseinrichtung eingerichtet, in Abhängigkeit einer Kraft, einer Bewegung, einer Position und/oder einer Zeit den Bewegungsfreiheitsgrad der Stativvorrichtung einzustellen. Die Steuerungseinrichtung kann dazu auch eine Uhr, einen Timer oder einen Zeitgeber aufweisen.

Die Stativvorrichtung weist ein Tragsystem umfassend eine Abstützeinrichtung zum ortsfesten Positionieren der Stativvorrichtung im Operationssaal auf, wobei der mindestens eine Tragarm in einem Lager und/oder Gelenk relativ zur Abstützeinrichtung bewegbar gelagert sein kann. Dabei ist der mindestens eine Tragarm in einem Aktionsradius in Abhängigkeit eines Bewegungsfreiheitsgrads des Tragsystems verlagerbar. Der oder die Bewegungsfreiheitsgrade und damit der Aktionsradius können z.B. durch die Art und Anzahl der Lager oder Gelenke definiert werden. Die Stativvorrichtung kann dabei von unterschiedlichen Angriffspunkten aus bedient und verlagert werden. Die Stativvorrichtung kann von alle oder beliebigen Angriffspunkten an der Stativvorrichtung verlagert werden, sei es an der medizintechnischen Einrichtung oder an einem der Tragarme. Irgendein an einer einzelnen Position angeordneter Bedienknopf muss nicht gesucht und betätigt werden. Somit wird eine "blinde" Bedienung und Verlagerung ermöglicht, also eine Bedienung, die kaum Aufmerksamkeit des Bedieners erfordert, was insbesondere im Zusammenhang mit schwierigen oder lebensbedrohlichen Operationen Vorteile liefert. Ein Operateur kann sich besser auf die eigentliche (Operations-)Tätigkeit konzentrieren.

Mit anderen Worten: Die Stativvorrichtung ist eingerichtet, einen Bewegungsfreiheitsgrad in Reaktion auf eine Bedienerbetätigung einzustellen, d.h. in Reaktion auf eine externe Krafteinwirkung. Bevorzugt ist die Steuerungseinrichtung eingerichtet, den Bewegungsfreiheitsgrad ausschließlich in Abhängigkeit von Kraft- und/oder Bewegungs-Messwerten der mindestens einen Sensoreinrichtung einzustellen. Bevorzugt ist die Stativvorrichtung autonom abbremsbar oder blockierbar oder loslösbar, d.h. allein in Abhängigkeit einer Krafteinwirkung durch den Bediener.

Das Erfassen einer externen Kraft kann auch das Erfassen einer Bewegung umfassen, insbesondere da bei einer festgestellten Vorrichtung eine von extern hervorgerufene Bewegung (zumindest eine soeben noch messbare Relativbewegung) eine externe Kraft erfordert. Ebenso kann das Erfassen einer Bewegung das Erfassen einer Kraft umfassen. Das Erfassen einer externen Kraft kann auch durch Erfassen eines Drehmoments erfolgen. Das Erfassen einer Kraft kann alternativ oder zusätzlich auch durch Erfassen eines Impulses erfolgen. Bevorzugt ist die Steuerungseinrichtung eingerichtet, die mindestens eine Bremse in Abhängigkeit eines externen Impulses oder einer Impulsänderung zumindest graduell zu lösen und/oder zumindest graduell festzustellen. Die Auswertung eines Impulses oder einer Impulsänderung kann das Positionieren der medizintechnischen Einrichtung in Abhängigkeit von oder in Reaktion auf eine sich ändernde Betätigung durch einen Bediener erleichtern.

Das Bremsen kann z.B. auf pneumatische, elektromagnetische und/oder friktionsbehaftete (mechanische) Weise erfolgen, insbesondere selektiv in einzelnen Gelenken oder Lagern.

Als Stativvorrichtung ist dabei bevorzugt eine Vorrichtung zum Halten, ortsfesten Anordnen und/oder Verlagern mindestens einer medizintechnische Einrichtung zu verstehen, die an einer Wand (in einem Wandlager) oder einer Decke oder auch am Boden eines Operationssaals oder irgendeines anderen Raumes für medizinische Zwecke fest montiert oder positioniert werden kann, also z.B. ein Deckenstativ. Die Stativvorrichtung ist dann nicht vollkommen frei im Operationssaal verlagerbar, sondern kann nur in einem bestimmten Aktionsradius verlagert werden, insbesondere relativ zu einem an einer Decke oder Wand des Operationssaals angeordneten Befestigungspunkt bzw. Montagepunkt. Die Stativvorrichtung umfasst nicht notwendigerweise eine medizintechnische Einrichtung, sondern kann auch lediglich durch ein Tragsystem gebildet sein. Die Stativvorrichtung kann als an einer Decke montierte Deckenversorgungseinheit ausgebildet sein und eine oder mehrere Versorgungskonsolen aufweisen, welche an einem oder zwei Tragarmen gelagert und positionierbar ist. Die Stativvorrichtung kann auch als Monitorträger ausgebildet sein. Die Stativvorrichtung kann auch als so genannter, insbesondere an einer Wand montierter Federarm ausgebildet sein und z.B. eine Leuchte aufweisen. Die Stativvorrichtung kann auch als so genannte, insbesondere an einer Decke montierte Zentralachse ausgebildet sein und eine Mehrzahl von Tragsystemen mit jeweils mindestens einem Träger aufweisen, an welchem z.B. ein Monitor oder eine Leuchte gelagert ist. Bevorzugt weist die Stativvorrichtung mindestens zwei Tragarme auf, an welchen jeweils mindestens eine Bremse der Bremseinrichtung vorgesehen ist.

Es sei darauf hingewiesen, dass der Anwendungsbereich "im Operationssaal" nur beispielhaft genannt ist, und dass die Stativvorrichtung ebenso auch z.B. im Zusammenhang mit Intensivmedizin, Diagnostikeinrichtungen oder nicht zuletzt auch mobilen Kranken- oder Rettungswagen angewendet werden kann.

Die Stativvorrichtung muss dabei nicht notwendigerweise fest an einer Wand oder Decke montiert sein, sondern kann auch auf einem fahrbaren Unterbau montiert sein. Der fahrbare Unterbau kann z.B. mittels Bremsen ortsfest im Raum positioniert werden. Mit anderen Worten: die Abstützeinrichtung kann einerseits eine Montageeinrichtung zur Wand- oder Deckenmontage sein (z.B. ein Deckenflansch), andererseits kann die Abstützeinrichtung auch ein fahrbarer Unterbau oder ein Fuß oder eine Bodenabstützung sein. Der fahrbare Unterbau lässt sich dann bevorzugt derart festsetzen oder parken, dass eine Krafteinwirkung auf einen der Tragarme nicht zu einer Verlagerung der Abstützeinrichtung relativ zum Boden führt. Der fahrbare Unterbau weist dann bevorzugt Rollen auf, welche mit Bremsen und wahlweise auch mit Antrieben zusammenwirken. Die Abstützeinrichtung kann z.B. durch einen Flansch oder irgendeine andere Schnittstelle gebildet sein, womit das Tragsystem an einer zumindest annähernd horizontal ausgerichteten Zimmerdecke oder auch einer zumindest annähernd vertikal ausgerichteten Wand montiert werden kann.

Als medizintechnische Einrichtung ist dabei bevorzugt eine Versorgungskonsole zu verstehen, mittels welcher Mittel für eine Versorgung eines Patienten und/oder Instrumente für einen Operateur und/oder Licht, Reinluft oder andere im Operationssaal benötigte Medien bereitgestellt werden können. Die medizintechnische Einrichtung weist bevorzugt irgendein Bedienpanel und/oder irgendeine Anzeigevorrichtung zum grafischen Darstellen von z.B. Patientendaten auf. Die medizintechnische Einrichtung kann wahlweise auch durch einen Monitor oder ein beliebiges anderes Endgerät bereitgestellt werden.

Als Tragarm ist dabei bevorzugt ein Ausleger oder Träger zu verstehen, welcher sich in einer bestimmten Richtung erstreckt und den gewünschten Aktionsradius für die unterschiedlichen Soll-Positionen der medizintechnischen Einrichtung sicherstellen kann, insbesondere durch eine Drehbewegung um ein Drehgelenk. Der Tragarm kann auch eine teleskopische Vorrichtung mit einem (zusätzlichen) Bewegungsfreiheitsgrad in translatorischer Richtung entlang der Längsachse des Tragarms sein. Der Tragarm hält nicht notwendigerweise eine medizintechnische Einrichtung, sondern kann auch als einer von mehreren Auslegern zwischen einzelnen Tragarmen angeordnet sein. In vielen Fällen weist die Stativvorrichtung einen oder zwei Tragarme/Ausleger auf.

Als Bremseinrichtung ist dabei bevorzugt eine Einrichtung zu verstehen, welche eingerichtet ist, mindestens einen Bewegungsfreiheitsgrad, bevorzugt mehrere und wahlweise alle Bewegungsfreiheitsgrade der Stativvorrichtung oder der medizintechnischen Einrichtung zu definieren, sei es "einzufrieren" oder festzusetzen oder freizugeben. Das Definieren der Freiheitsgrade kann dabei zeitbezogen erfolgen, also z.B. Lösen einer Bremse für einen Zeitraum von z.B. 5sec. Die Bremseinrichtung kann z.B. mindestens eine Bremse umfassen, welche eine Funktion im Sinne einer Feststellbremse erfüllen kann.

Als "externe Kraft" ist dabei bevorzugt eine Kraft zu verstehen, welche von einem Bediener von extern aktiv auf das System ausgeübt wird. Eine externe Kraft umfasst z.B. keine Trägheitskräfte. Eine externe Kraft kann dabei auch nicht durch von irgendwelche Motoren oder Antrieben des Systems ausgeübten Kräften bereitgestellt werden. Vielmehr ist die externe Kraft als eine Kraft zu verstehen, die vollständig entkoppelt vom System aufgebracht wird.

Der Bewegungsfreiheitsgrad des Tragsystems bzw. der medizintechnischen Einrichtung kann mehrere Freiheitsgrade umfassen, also z.B. translatorische und/oder rotatorische Freiheitsgrade in mehreren Ebenen bzw. um mehrere Achsen. Dabei kann nicht nur ein autonomes Lösen einer Bremse in Reaktion auf eine externe Kraft erfolgen. Vielmehr kann gemäß weiterer Merkmale z.B. auch die Position des Tragarms relativ zur Abstützeinrichtung oder relativ zu einem weiteren Tragarm festgelegt werden, insbesondere ab einem Moment, zu welchem ein Benutzer keine Kraft mehr auf den Tragarm ausübt. Mit anderen Worten kann die Stativvorrichtung auch selbstpositionierend ausgestaltet werden. Die Stativvorrichtung kann sich autonom positionieren.

Gemäß einem Ausführungsbeispiel ist die Steuerungseinrichtung eingerichtet, die mindestens eine Bremse in Abhängigkeit des Betrags oder der Richtung der externen Kraft und/oder der Richtung der Bewegung und/oder des Weges der Bewegung zumindest graduell zu lösen oder auch festzustellen. Hierdurch kann ein gewünschtes Verlagern/Positionieren in die Soll-Position effizient oder effektiv erfolgen. Bevorzugt wird die externe Kraft (Betrag, Richtung) dabei jedenfalls ausgewertet. Falls jedoch keine externe Kraft mehr ausgeübt wird, kann auch allein eine Bewegung des Systems ausgewertet werden. Eine solche Bewegung beruht dann z.B. auf Massenträgheit und kann gestoppt oder zumindest graduell verlangsamt werden.

Bevorzugt ist die Steuerungseinrichtung eingerichtet, die Bremseinrichtung in Abhängigkeit der von der mindestens einen Sensoreinrichtung erfassten externen Kraft zumindest graduell zu lösen. Das Lösen oder Freigeben in Abhängigkeit der externen Kraft ermöglicht ein autonomes Positionieren oder eine Selbstjustage der Stativvorrichtung, ohne dass ein Bediener eine Taste betätigen muss oder irgendeine bestimmte Bedienereingabe vornehmen muss. Der Bediener braucht die Stativvorrichtung oder medizintechnische Einrichtung lediglich anzufassen und einen Druck in die gewünschte Richtung auszuüben.

Bevorzugt ist die Steuerungseinrichtung eingerichtet, die mindestens eine Bremse in Abhängigkeit der von der mindestens einen Sensoreinrichtung erfassten Bewegung, insbesondere der Richtung und/oder der Geschwindigkeit, festzustellen oder zumindest graduell fester zu stellen. Das Feststellen in Abhängigkeit der Bewegung ermöglicht ein selbständiges Positionieren in einer Sollposition.

Bevorzugt umfasst die Stativvorrichtung die medizintechnische Einrichtung, wobei die medizintechnische Einrichtung z.B. an einem Ende des Tragsystems unterhalb des Tragsystems angeordnet ist. Dabei kann auch der medizintechnischen Einrichtung eine spezifische Bremse der Bremseinrichtung zugeordnet sein.

Gemäß einem Ausführungsbeispiel weist die medizintechnische Einrichtung auf, wobei die Bremseinrichtung mehrere Bremsen umfasst, welche jeweils an einem jeweiligen Tragarm und/oder an der medizintechnischen Einrichtung angeordnet sind, wobei die Steuerungseinrichtung eingerichtet ist, die Bremseinrichtung selektiv in Bezug auf den mindestens einen Tragarm und/oder in Bezug auf die medizintechnische Einrichtung einzustellen, insbesondere in Abhängigkeit der Richtung der externen Kraft. Hierdurch kann das Positionieren auf genauere Weise erfolgen. Insbesondere muss die externe Kraft von einem Bediener nur in einer Richtung aufgebracht werden, selbst bei einer Vielzahl von Tragarmen oder Gelenken. Nichtsdestotrotz kann die externe Kraft in Hinblick auf eine Vielzahl von Richtungen ausgewertet werden und der Bewegungsfreiheitsgrad richtungsabhängig eingestellt werden, insbesondere in Verbindung mit einer Vielzahl von richtungssensiblen Kraft- und/oder Bewegungssensoren. Bevorzugt ist die medizintechnische Einrichtung am mindestens einen Tragarm relativ zum Tragarm bewegbar gelagert.

Gemäß einem Ausführungsbeispiel ist die Steuerungseinrichtung eingerichtet, die mindestens eine Bremse in Abhängigkeit eines von einer Positionssensoreinrichtung erfassten Positionssignals einzustellen, insbesondere bei Stillstand der Stativvorrichtung, bevorzugt bei Stillstand nach einer vordefinierten Mindestdauer. Dies ermöglicht z.B. ein automatisches Feststellen der Bremse(n), sobald die Stativvorrichtung vom Bediener in einer Sollposition gehalten wird, insbesondere für eine Mindestdauer von z.B. 1sec. oder 2sec. gehalten wird. Die vordefinierte Mindestdauer liegt dabei bevorzugt im Bereich von 0.5 bis 3sec. Die Positionssensoreinrichtung kann dabei durch eine der Sensoreinrichtungen bereitgestellt werden, z.B. in Form eines GPS- oder DGPS-Empfängers oder auch in Form eines Beschleunigungssensors.

Gemäß einem Ausführungsbeispiel weist die Stativvorrichtung mindestens ein manuell betätigbares Funktionselement auf, insbesondere ein Tablett, an welchem eine Sensoreinrichtung der mindestens einen Sensoreinrichtung angeordnet ist, insbesondere mindestens ein Kraftsensor. Eine Betätigung des Tabletts kann auf intuitive und gut zugängliche Weise erfolgen.

Gemäß einem Ausführungsbeispiel umfasst die Stativvorrichtung die medizintechnische Einrichtung und weist eine Montage-Schnittstelle auf, in welcher die externe Kraft erfassbar ist, wobei die Montage-Schnittstelle an der medizintechnischen Einrichtung vorgesehen ist, und wobei in der Montage-Schnittstelle ein erstes Funktionselement, insbesondere in Form eines Griffs oder einer Stange, befestigt ist. Die Montage-Schnittstelle ermöglicht das Erfassen einer Kraft oder auch eines Moments auf einfache Weise, insbesondere in eine Vielzahl von Richtungen.

Gemäß einem Ausführungsbeispiel weist die Stativvorrichtung eine insbesondere von außen zugängliche Montage-Schnittstelle auf, in welcher die externe Kraft erfassbar ist, und in welcher mindestens ein von der Bremseinrichtung entkoppeltes manuell betätigbares Funktionselement befestigt ist, und an welcher in einem Befestigungs- oder Montagepunkt eine Sensoreinrichtung der mindestens einen Sensoreinrichtung angeordnet ist, insbesondere mindestens ein Kraftsensor. Mittels des Funktionselements kann die externe Kraft erfasst werden. Es hat sich gezeigt, dass die Bedienung stark dadurch vereinfacht werden kann, wenn auf jegliche zusätzliche Elemente verzichtet wird, also wenn eine Kraft an denjenigen Schnittstellen gemessen wird, an welchen ein Bediener (ohne den Zweck, eine Bremse zu lösen) ohnehin üblicherweise oder aus Gewohnheit angreift. Eine von außen zugängliche Montage-Schnittstelle ist dabei für einen Bediener derart von außen zugänglich, dass an der Montage-Schnittstelle eine externe Kraft in die Stativvorrichtung einleitbar ist.

Dabei kann eine Bedienung der Stativvorrichtung, insbesondere eine Krafteinwirkung auf die Stativvorrichtung, erfasst und ausgewertet werden, ohne dass ein Bediener eine separate Taste betätigen muss. Der Bediener kann die gesamte Stativvorrichtung durch eine Krafteinwirkung über das Funktionselement (insbesondere auf ein Tablett) in eine Soll-Position verlagern, insbesondere einhändig. Über das Tablett kann dabei auf einfache Weise auch eine Drehung der medizintechnischen Einrichtung bewirkt werden, insbesondere da das Tablett einen großen Hebel ermöglicht. Ein Operateur kann das Tablett dabei mehr oder weniger "blind" in die Nähe einer Operationsstelle schieben, z.B. um Instrumente unmittelbar neben einer Eingriffs-Stelle (z.B. Implantations-Stelle) am Patienten bereitzustellen, z.B. über dem Bauch des Patienten, oder neben der Hüfte oder dem Knie des Patienten. Indem die Montage-Schnittstelle derart (insbesondere von außen zugänglich) angeordnet ist, dass in der Montage-Schnittstelle eine Krafteinwirkung von außen erfassbar ist, kann an der Stativvorrichtung ein Funktionselement, insbesondere ein mechanisches, nicht elektronisches Funktionselement befestigt werden, mittels welchem die Stativvorrichtung positioniert oder verlagert werden kann, ohne dass eine Betätigung eines Schalters erforderlich ist.

Das mindestens eine Funktionselement kann dabei wahlweise an mindestens einem Tragarm und/oder an der medizintechnischen Einrichtung angeordnet sein. Bevorzugt weist die Stativvorrichtung die medizintechnische Einrichtung auf, wobei das Funktionselement in einem Befestigungs- oder Montagepunkt an der medizintechnischen Einrichtung befestigt ist.

Das mindestens eine Funktionselement kann dabei auch mit einem gewissen Spiel gelagert sein, insbesondere nachgiebig und/oder in Abhängigkeit von einer Mindestkraft, und insbesondere derart, dass ein Bediener beim Betätigen des Funktionselements spürt, dass eine Betätigung als Benutzereingabe erfasst wurde. Mit anderen Worten: Das mindestens eine Funktionselement ist dann relativ zur Versorgungskonsole verlagerbar, zumindest im Bereich von einigen Winkeln (z.B. 10-20°) oder einigen Millimetern oder Zentimetern, und kann eingerichtet sein, dem Bediener ein insbesondere haptisches feedback zu geben. Das mindestens eine Funktionselement kann dazu bewegungstolerant an der Stativvorrichtung gelagert sein.

Als von der Bremseinrichtung entkoppeltes Funktionselement ist dabei bevorzugt eine Einrichtung zu verstehen, welche für sich betrachtet funktionell nicht in Verbindung mit der Bremseinrichtung steht. Mit anderen Worten: Das Funktionselement erfüllt eine Funktion ungleich einer Betätigung der Bremseinrichtung. Das Funktionselement ist bevorzugt eine Einrichtung, welche manuell ergriffen werden kann, z.B. ein Griff oder eine Stange, oder ein Tablett, z.B. zur Ablage von Instrumenten. Bevorzugt ist das Funktionselement derart an der Stativvorrichtung angeordnet, dass ein Bediener es in einer stehenden oder sitzenden Position gut erreichen kann. Das Funktionselement kann z.B. an einer Unterseite oder im unteren Bereich der medizintechnischen Einrichtung angeordnet sein.

Gemäß einem Ausführungsbeispiel umfasst die Stativvorrichtung die medizintechnische Einrichtung, wobei die Montage-Schnittstelle an der medizintechnischen Einrichtung vorgesehen ist, und wobei in der Montage-Schnittstelle ein erstes Funktionselement, insbesondere in Form eines Griffs oder einer Stange, befestigt ist. Indem die Kraft oder Bewegung an einem Funktionselement erfasst wird, welches dem Bediener ohnehin in seiner Lage und Erreichbarkeit bekannt ist, kann die Verlagerung der Stativvorrichtung besonders einfach und intuitiv erfolgen. Bevorzugt ist am ersten Funktionselement ein zweites Funktionselement befestigt, insbesondere in Form eines Tabletts. Bei dieser Anordnung kann wahlweise auf das erste oder zweite Funktionselement eine Kraft ausgeübt werden. Dies erhöht die Flexibilität bei der Bedienung oder auch die Ergonomie, insbesondere in Verbindung mit spezifischen Haltungen und Positionen eines Operateurs. Dabei kann die Bedieneingabe bei beiden Funktionselementen auf dieselbe Art und Weise erfolgen und auf dieselbe Art und Weise ausgewertet werden, so dass es für den Bediener nicht erforderlich ist, zwischen den Funktionselementen zu unterscheiden.

Gemäß einem Ausführungsbeispiel weist die Stativvorrichtung einen bevorzugt für den Bediener sichtbaren oder als solchen gekennzeichneten Betätigungsbereich auf, welcher von außen für den Bediener zugänglich ist und an welchem die externe Kraft in die Stativvorrichtung einleitbar ist. Der Betätigungsbereich kann z.B. durch einen Seitenflächenabschnitt eines Tragarms oder durch eine Seite einer/der medizintechnischen Einrichtung gebildet sein, oder durch irgendein freies Ende eines Tragarms oder einer/der medizintechnischen Einrichtung. Der Betätigungsbereich kann dabei auch als solcher markiert sein, sei es durch farbliche Hervorhebungen und/oder durch eine strukturierte Oberfläche, insbesondere zwecks haptischer Erkennung des Betätigungsbereichs (ohne visuellen Kontakt). Der Betätigungsbereich kann z.B. durch ein Verkleidungsteil eines Tragarms oder einer medizintechnischen Einrichtung bereitgestellt sein. Bevorzugt ist im Betätigungsbereich kein Funktionselement angeordnet.

Im Betätigungsbereich oder unter dem Betätigungsbereich kann wenigstens eine der Sensoreinrichtungen angeordnet sein. Der Betätigungsbereich kann dabei auch hinterleuchtet sein, z.B. mittels einer insbesondere dimmbaren LED oder einer bevorzugt vergleichsweise schwach leuchtenden Flächenbeleuchtung, also bevorzugt einem Flächenstrahler anstatt einem Punktstrahler.

Der Betätigungsbereich wird bevorzugt von einer ohnehin vorhandenen Komponente der Stativvorrichtung bereitgestellt, z.B. von einem Verkleidungsteil. Im Betätigungsbereich ist bevorzugt kein Taster angeordnet, obgleich auch ein (Zentral-)Taster, insbesondere ein einziger Taster für alle Bremsen, vorgesehen sein kann. Der Taster kann auf einfache Weise ermöglichen, der Steuerungseinrichtung zu signalisieren, dass keine Fehlbedienung vorliegt, also dass insbesondere nicht aus Versehen eine externe Kraft auf die Stativvorrichtung ausgeübt wurde.

Gemäß einem Ausführungsbeispiel ist die mindestens eine Sensoreinrichtung für Dauerbetrieb eingerichtet, wobei ein Messwert der externen Kraft oder Bewegung unabhängig vom Betriebszustand der Stativvorrichtung erfassbar ist und unabhängig vom Betriebszustand ausgebbar ist. Hierdurch kann sichergestellt werden, dass die Sensoreinrichtung eine externe Kraft oder Bewegung unabhängig von einem Betriebszustand der Stativvorrichtung erfassen kann, also z.B. unabhängig davon, ob eine medizintechnische Einrichtung der Stativvorrichtung betrieben wird. Dies ermöglicht eine Verlagerung der Stativvorrichtung durch den Bediener selbst dann, wenn die Stativvorrichtung oder die medizintechnische Einrichtung nicht eingeschaltet ist oder aktuell nicht im Zusammenhang mit irgendeiner Operation verwendet wird.

Bevorzugt weist die mindestens eine Sensoreinrichtung eine Energiequelle auf und/oder ist unabhängig von der Steuerungseinrichtung an eine Energiequelle gekoppelt. Hierdurch kann sichergestellt werden, dass die Sensoreinrichtung eine externe Kraft oder Bewegung unabhängig von einem Betriebszustand der Steuerungseinrichtung erfassen kann und die Steuerungseinrichtung z.B. auch aus einem standby-Zustand aufwecken kann, um eine Bremseinrichtung in Reaktion auf eine externe Krafteinwirkung einstellen zu können.

Gemäß einem Ausführungsbeispiel weist die Stativvorrichtung mindestens einen in Verbindung mit der Steuerungseinrichtung stehenden Antrieb zum motorischen Bewegen mindestens eines Tragarms auf, wobei die Steuerungseinrichtung eingerichtet ist, die durch eine externe Kraft hervorgerufene Bewegung in Abhängigkeit eines Betriebszustands des mindestens einen Antriebs auszuwerten. Hierdurch kann eine durch einen Bediener hervorgerufene Bewegung von einem durch den Antrieb hervorgerufene Bewegung unterschieden werden.

Gemäß einem Ausführungsbeispiel ist die Stativvorrichtung autonom ungebremst, wobei die Steuerungseinrichtung eingerichtet ist, die mindestens eine Bremse in Abhängigkeit der von der mindestens einen Sensoreinrichtung erfassten externen Kraft zu lösen oder zumindest graduell freizugeben, wenn der Betrag der externen Kraft einen vorbestimmten Schwellwert überschreitet oder (insbesondere in einem vorbestimmten Maße) steigt. Hierdurch kann automatisch ein Freigeben einer oder mehrerer Bremsen erfolgen, insbesondere in dem Moment, in welchem ein Bediener eine Kraft auf den Tragarm oder die medizintechnische Einrichtung ausübt. Der Bediener muss dabei keinen zusätzlichen Tasten oder Schalter betätigen.

Als eine "autonom ungebremste" Stativvorrichtung ist dabei bevorzugt eine Stativvorrichtung zu verstehen, bei welcher nicht notwendigerweise ein mechanischer oder elektrischer Schalter oder Taster betätigt werden muss, um eine Bremse der Stativvorrichtung zu lösen und/oder die Stativvorrichtung bzw. die medizintechnische Einrichtung verlagern zu können. Eine solche Stativvorrichtung kann auch als "autonom bewegungsfrei" beschrieben werden. Eine solche Stativvorrichtung kann eine "autonom selbstlösende" oder autonom entkoppelnde oder autonom freigebende Bremseinrichtung aufweisen. Der Begriff "autonom" beinhaltet dabei, dass eine weitere Bedienereingabe nicht erforderlich ist. Es ist ausreichend, die externe Kraft aufzubringen. Eine Verlagerung kann in Reaktion auf eine externe Kraft erfolgen, insbesondere in Richtung der Kraft.

Gemäß einem Ausführungsbeispiel ist die mindestens eine Sensoreinrichtung eingerichtet, zeitbezogen einen Kraftverlauf zu erfassen, wobei die Steuerungseinrichtung eingerichtet ist, die mindestens eine Bremse in Abhängigkeit der von der mindestens einen Sensoreinrichtung erfassten externen Kraft als Funktion des Kraftverlaufs graduell freizugeben, insbesondere bei stärker ansteigender Kraft schneller zu lösen und/oder bei stärker sinkender Kraft schneller festzusetzen. Hierdurch kann die Soll-Position auf intuitive Weise angefahren werden. Eine Änderung der vom Bediener aufgebrachten Kraft kann zum Einstellen der Bremse(n) ausgewertet werden.

Gemäß einem Ausführungsbeispiel ist die mindestens eine Sensoreinrichtung eingerichtet, eine Kraftrichtung zu erfassen, wobei die Steuerungseinrichtung eingerichtet ist, die mindestens eine Bremse in Abhängigkeit der von der mindestens einen Sensoreinrichtung erfassten Kraftrichtung freizugeben. Hierdurch können selektiv diejenigen Bremsen freigegeben werden, welche eine Verlagerung in der gewünschten Richtung ermöglichen. Hierdurch kann sichergestellt werden, dass die medizintechnische Einrichtung nur in einer bestimmten gewünschten Richtung verlagert wird, z.B. weg vom Patienten.

Gemäß einer Variante ist die Stativvorrichtung selbstpositionierend, wobei die Steuerungseinrichtung eingerichtet ist, die mindestens eine Bremse in Abhängigkeit der von der mindestens einen Sensoreinrichtung erfassten externen Kraft festzustellen oder zumindest graduell fester zu stellen, wenn der Betrag der externen Kraft einen vorbestimmten Schwellwert unterschreitet oder (insbesondere in einem vorbestimmten Maße) sinkt, und/oder die mindestens eine Bremse in Abhängigkeit der von der mindestens einen Sensoreinrichtung erfassten Bewegung festzustellen oder zumindest graduell fester zu stellen, wenn die Bewegung eine vorbestimmte Weglänge überschreitet oder wenn die Geschwindigkeit der Bewegung einen vorbestimmten Schwellwert unterschreitet oder (insbesondere in einem vorbestimmten Maße) sinkt. Hierdurch kann automatisch eine Soll-Position gefunden werden, insbesondere in dem Moment, in welchem ein Bediener den Tragarm nicht mehr verlagert. Durch Auswerten einer Bewegungsgeschwindigkeit kann dabei auch erfasst werden, ob der Bediener den Tragarm noch verschiebt oder nicht, insbesondere dann, wenn die mindestens eine Bremse eine Reaktionskraft sicherstellt, welche bewirkt, dass die Bewegung des Tragarms sofort abgebremst wird, wenn der Tragarm nicht mehr durch eine externe Krafteinwirkung aktiv durch einen Bediener verlagert wird.

Gemäß einem Ausführungsbeispiel ist der mindestens eine Tragarm und/oder die medizintechnische Einrichtung translatorisch verlagerbar in einem Linearlager und/oder schwenkbar in einem Drehgelenk gelagert, wobei jeweils mindestens eine Sensoreinrichtung in einem jeweiligen Gelenk oder Lager angeordnet sein kann.

Gemäß einer Variante ist der mindestens eine Tragarm und/oder die medizintechnische Einrichtung translatorisch verlagerbar in einem Linearlager gelagert, wobei die mindestens eine Sensoreinrichtung am oder im Linearlager angeordnet ist und eingerichtet ist, eine im Linearlager übertragene Kraft zu erfassen, wobei die Steuerungseinrichtung zum Auswerten des Betrags der Kraft eingerichtet ist, insbesondere in Abhängigkeit eines vordefinierbaren Schwellwertes.

Gemäß einer Variante ist der mindestens eine Tragarm schwenkbar in einem Drehgelenk gelagert, wobei die mindestens eine Sensoreinrichtung am Drehgelenk angeordnet ist und eingerichtet ist, ein im Drehgelenk übertragenes Drehmoment zu erfassen, wobei die Steuerungseinrichtung zum Auswerten des Betrags des Drehmoments eingerichtet ist, insbesondere in Abhängigkeit eines vordefinierbaren Schwellwertes.

Gemäß einer Variante ist die mindestens eine Sensoreinrichtung am mindestens einen Tragarms angeordnet, insbesondere an einer seitlichen (beispielsweise zumindest annähernd vertikal ausgerichteten) Fläche des Tragarms, und eingerichtet, eine Richtung und/oder einen Weg einer Bewegung des Tragarms zu erfassen, wobei die Steuerungseinrichtung zum Auswerten der Richtung und/oder des Wegs in Bezug auf die Abstützeinrichtung oder mindestens einen weiteren Tragarm eingerichtet ist. Bevorzugt ist die Steuerungseinrichtung eingerichtet, die Bremseinrichtung in Abhängigkeit der Richtung der externen Kraft oder Bewegung einzustellen.

Bevorzugt ist die Bremseinrichtung mittels der Steuerungseinrichtung derart ansteuerbar, dass eine Bewegung des Tragsystems in Reaktion auf eine Krafteinwirkung auf eine der Komponenten der Stativvorrichtung zulässig/möglich ist.

Gemäß einem Ausführungsbeispiel ist mindestens eine der mindestens einen Sensoreinrichtung ein Bewegungssensor, welcher eingerichtet ist, eine Bewegung in Bezug auf absolute Raumkoordinaten zu erfassen, insbesondere in allen drei Raumrichtungen, insbesondere ein Bewegungssensor aus der Gruppe Radarsensor, Beschleunigungssensor, optischer Sensor, auf terrestrischer Kopplung basierender Peilsensor.

Bevorzugt weist die Stativvorrichtung eine Positionssensoreinrichtung auf, welche mit der Steuerungseinrichtung verbunden ist und eingerichtet ist, eine absolute Position eines (jeweiligen) Tragarms und/oder die absolute Position der medizintechnischen Einrichtung zu erfassen. Hierdurch kann die Steuerungseinrichtung auch absolute Positionsdaten berücksichtigen, z.B. um Kollisionen mit anderen Objekten zu vermeiden. Die Positionssensoreinrichtung kann dabei eine Mehrzahl von Positionssensoren umfassen, welche jeweils an bestimmten Positionen am jeweiligen Tragarm oder an der medizintechnischen Einrichtung angeordnet sein können. Ein jeweiliger Positionssensor kann z.B. in Form eines GPS-Sensors oder DGPS-Sensors bereitgestellt werden.

Gemäß einem Ausführungsbeispiel ist wenigstens eine Sensoreinrichtung der mindestens einen Sensoreinrichtung in einem Drehgelenk der Stativvorrichtung angeordnet. Im Drehgelenk kann eine Kraft oder Bewegung, insbesondere die Richtung einer Kraft oder Bewegung, auf sehr genaue Weise erfasst werden, insbesondere basierend auf relativen Bewegungen und ohne Bezug zu einer absoluten Position. Dabei kann auch bei einer Vielzahl von Tragarmen die Kraft/Bewegung erfasst werden, insbesondere unabhängig davon, an welcher Komponente der Stativvorrichtung die externe Kraft aufgebracht wird.

Gemäß einem Ausführungsbeispiel ist die mindestens eine Sensoreinrichtung ein Kraftsensor aus der Gruppe Drucksensor, Dehnungsmessstreifen, Torsionssensor. Gemäß einer Variante kann der Kraftsensor dabei auch eine Bewegung oder eine Kraftänderung (insbesondere mit Zeitbezug) erfassen und als Impulssensor ausgebildet sein, oder als Drehmomentsensor ausgebildet sein.

Gemäß einer Variante weist die Stativvorrichtung mindestens eine Anzeigeeinrichtung zum Anzeigen einer zu einer Kollision mit dem Hindernis führenden Relativbewegung auf, welche mit der Steuerungseinrichtung verbunden ist.

Offenbart (nicht beansprucht) wird auch eine Stativvorrichtung zur Anordnung im Operationssaal und zum örtlichen Verlagern einer medizintechnischen Einrichtung, umfassend eine Bremseinrichtung mit mindestens einer Bremse, die eingerichtet ist, einen Bewegungsfreiheitsgrad der medizintechnischen Einrichtung einzustellen; wobei die Stativvorrichtung ferner umfasst: eine mit der Bremseinrichtung in Verbindung stehende Steuerungseinrichtung eingerichtet zum Auswerten einer auf die Stativvorrichtung einwirkenden externen Kraft oder einer durch die externe Kraft hervorgerufenen Bewegung und eingerichtet zum Ansteuern der Bremseinrichtung und zum Einstellen des Bewegungsfreiheitsgrads. Hierdurch ergeben sich bereits zuvor erläuterte Vorteile.

Offenbart (nicht beansprucht) wird auch eine Steuerungseinrichtung für eine medizintechnische Stativvorrichtung, insbesondere für eine erfindungsgemäße Stativvorrichtung, wobei die Steuerungseinrichtung eingerichtet ist zum Auswerten einer auf die Stativvorrichtung einwirkenden externen Kraft oder einer durch die externe Kraft hervorgerufenen Bewegung und eingerichtet ist, eine Bremseinrichtung der Stativvorrichtung in Abhängigkeit der Kraft oder Bewegung anzusteuern, insbesondere zu lösen, und dadurch einen Bewegungsfreiheitsgrad der Stativvorrichtung einzustellen. Hierdurch ergeben sich bereits zuvor erläuterte Vorteile. Bevorzugt ist die Steuerungseinrichtung eingerichtet, den Bewegungsfreiheitsgrad in Abhängigkeit eines Positionssignals einer Positionssensoreinrichtung einzustellen.

Die zuvor genannte Aufgabe wird auch gelöst durch ein Verfahren zum automatischen Positionieren einer medizintechnischen Einrichtung an einer im Operationssaal anordenbaren zuvor beschriebenen erfindungsgemäßen Stativvorrichtung, gekennzeichnet durch die Schritte:
- Erfassen einer auf die Stativvorrichtung einwirkenden oder ausgeübten externen Kraft oder einer durch die externe Kraft hervorgerufenen Bewegung. Wahlweise kann auch die durch die externe Kraft hervorgerufene Bewegung des mindestens einen Tragarms, insbesondere einer Bewegung relativ zur Abstützeinrichtung, bevorzugt mittels mindestens einer Sensoreinrichtung der Stativvorrichtung;
- Ausgeben eines entsprechenden Messwerts an eine Steuerungseinrichtung der Stativvorrichtung;
- Auswerten des Messwerts mittels der Steuerungseinrichtung, insbesondere Auswerten des Betrages und/oder der Richtung der externen Kraft und wahlweise auch der Bewegung (Richtung, Betrag bzw. Weg der Bewegung); und
- Ansteuern einer Bremseinrichtung der Stativvorrichtung und Einstellen eines Bewegungsfreiheitsgrads der medizintechnischen Einrichtung bzw. der Stativvorrichtung mittels der Steuerungseinrichtung in Abhängigkeit des Messwerts, betreffend eine zeitliche Änderung, einen Betrag und/oder eine Richtung der externen Kraft, und/oder eine Richtung und/oder eine Geschwindigkeit der Bewegung, wobei die Bremseinrichtung mittels der Steuerungseinrichtung in Abhängigkeit des Messwerts autonom und ohne Betätigung eines zusätzlichen Schalters ausschließlich aufgrund der auf die Stativvorrichtung einwirkenden externen Kraft gelöst wird. Hierdurch ergeben sich zuvor bereits beschriebene Vorteile.

Das Ansteuern umfasst dabei bevorzugt das Lösen oder zusätzlich auch wieder Feststellen mindestens einer Bremse der Bremseinrichtung.

Wahlweise kann die durch die externe Kraft hervorgerufene Bewegung des mindestens einen Tragarms, insbesondere einer Bewegung relativ zur Abstützeinrichtung, erfasst werden.

Gemäß einer Variante kann dabei zusätzlich auch ein aktives, motorisches Positionieren der medizintechnischen Einrichtung erfolgen, insbesondere durch Ansteuern der Bremseinrichtung und/oder einer Antriebseinrichtung der Stativvorrichtung, jeweils mittels der Steuerungseinrichtung.

Gemäß einer Ausführungsform umfasst das Ansteuern der Bremseinrichtung ein zumindest graduelles Lösen mindestens einer Bremse der Bremseinrichtung, sobald eine externe Kraft erfasst wird oder wenn die externe Kraft erstmals einen bestimmten Schwellwert überschreitet oder in einer bestimmten Richtung ausgeübt wird. Beim zumindest graduellen Lösen kann der Bewegungsfreiheitsgrad situationsabhängig eingestellt werden. Hierdurch kann z.B. vermieden werden, dass Stöße oder ungewollte Impulse auf die Stativvorrichtung dazu führen, dass die Stativvorrichtung nicht mehr abgebremst/geparkt ist. Gemäß einer Variante wird mindestens eine Bremse der Bremseinrichtung graduell gelöst oder vollständig gelöst, sobald sich die externe Kraft vergrößert oder einen bestimmten Schwellwert überschreitet.

Gemäß einer Ausführungsform wird die Bremseinrichtung graduell fester gestellt oder festgestellt, sobald sich die externe Kraft vermindert oder keine externe Kraft mehr erfasst wird, oder sobald sich die Richtung der externen Kraft ändert, insbesondere ab einer Änderung der Richtung in Abhängigkeit von einem Mindestschwellwert, beispielsweise mindestens 30 oder 45 Grad. Die Richtungs-Steuerung ermöglicht, möglichst schnell auf eine geänderte Bedienung zu reagieren, so dass die medizintechnische Einrichtung möglichst schnell und ohne Umwege in eine Sollposition verlagert werden kann.

Gemäß einer Ausführungsform wird die Stativvorrichtung in einer festen Position positioniert, indem die Bremseinrichtung in Abhängigkeit der von der mindestens einen Sensoreinrichtung erfassten externen Kraft festgestellt wird, wenn der Betrag der externen Kraft einen vorbestimmten Schwellwert unterschreitet oder (insbesondere in einem vorbestimmten Maße) sinkt, und/oder indem die Bremseinrichtung in Abhängigkeit der von der mindestens einen Sensoreinrichtung erfassten Bewegung festgestellt wird, wenn die Bewegung eine vorbestimmte Weglänge überschreitet oder wenn die Geschwindigkeit der Bewegung einen vorbestimmten Schwellwert unterschreitet oder (insbesondere in einem vorbestimmten Maße) sinkt. Hierdurch kann die Stativvorrichtung in Reaktion auf ein Loslassen der Stativvorrichtung, insbesondere des Tabletts, positioniert werden. Das Loslassen der Stativvorrichtung, also das nicht mehr manuelle Verlagern (keine oder verringerte externe Kraft) kann dabei als ein Befehl ausgewertet werden, dass eine Soll-Position erreicht ist oder in Kürze erreicht sein wird.

Gemäß einer Ausführungsform wird die durch eine externe Kraft hervorgerufene Bewegung in Abhängigkeit eines Betriebszustands mindestens eines Antriebs der Stativvorrichtung ausgewertet. Hierdurch kann z.B. auch zumindest teilweise eine motorische Verlagerung mindestens eines Tragarms erfolgen, und gleichzeitig kann ein Bediener einen weiteren Tragarm der Stativvorrichtung positionieren.

Offenbart (nicht beansprucht) wird auch ein Verfahren zum automatischen Positionieren einer medizintechnischen Einrichtung einer im Operationssaal anordenbaren Stativvorrichtung, gekennzeichnet durch die Schritte: Erfassen einer auf die Stativvorrichtung einwirkenden externen Kraft oder einer durch die externe Kraft hervorgerufenen Bewegung; und Ansteuern einer Bremseinrichtung der Stativvorrichtung und Einstellen eines Bewegungsfreiheitsgrads der medizintechnischen Einrichtung bzw. der Stativvorrichtung mittels der Steuerungseinrichtung in Abhängigkeit des Messwerts, insbesondere in Abhängigkeit des Betrags und/oder der Richtung der externen Kraft. Hierdurch ergeben sich bereits zuvor erläuterte Vorteile.

Offenbart (nicht beansprucht) wird auch die Verwendung einer Steuerungseinrichtung zum Positionieren einer Stativvorrichtung im Operationssaal beim örtlichen Verlagern einer an der Stativvorrichtung montierten medizintechnischen Einrichtung, insbesondere zum Positionieren einer erfindungsgemäßen Stativvorrichtung, wobei die Steuerungseinrichtung mit wenigstens einer Bremseinrichtung sowie wenigstens einer Sensoreinrichtung eingerichtet zum Erfassen einer auf die Stativvorrichtung einwirkenden externen Kraft oder einer durch die externe Kraft hervorgerufenen Bewegung kommuniziert und die Bremseinrichtung in Abhängigkeit des Messwerts ansteuert und einen Bewegungsfreiheitsgrad der Stativvorrichtung einstellt. Diese Art der Steuerung erleichtert die Bedienung und ermöglicht einen hohen Grad an Intuition oder Ergonomie.

In den nachfolgenden Zeichnungsfiguren wird die Erfindung anhand von Ausführungsbeispielen näher erläutert. Es zeigen:
- Figur 1: in schematischer Darstellung in einer perspektivischen Seitenansicht eine Stativvorrichtung gemäß einem Ausführungsbeispiel der Erfindung in Verbindung mit einer medizintechnischen Einrichtung;
- Figur 2: in schematischer Darstellung in einer perspektivischen Seitenansicht die in der Figur 1 gezeigte medizintechnische Einrichtung; und
- Figur 3: in schematischer Darstellung Verfahrensschritte eines Verfahrens gemäß einzelnen Ausführungsformen der Erfindung.

Im Zusammenhang mit der Beschreibung einzelner Figuren wird bei Bezugszeichen, die nicht explizit erläutert werden, auf die weiteren Figuren verwiesen.

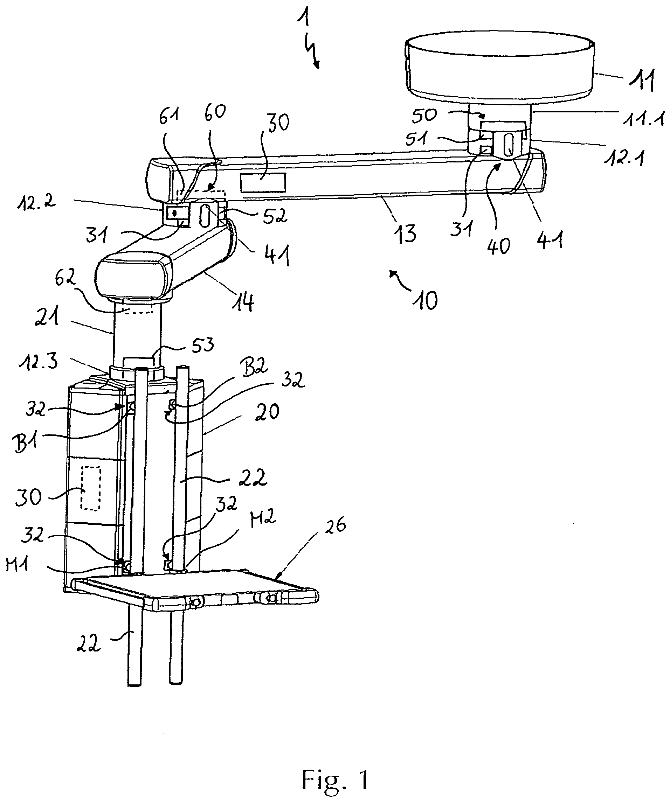

In der Figur 1 eine Deckenstativvorrichtung 1 mit einem Tragsystem 10 gezeigt, welches zwei Tragarme 13, 14 umfasst. Die Deckenstativvorrichtung 1 ist mittels einer Montageeinrichtung (Deckenflansch) 11 an einer Decke montierbar, wobei einer der Tragarme 13 über einen Trägerabschnitt, insbesondere über ein Säulenstück 11.1, mit der Montageeinrichtung 11 verbunden ist. Zwischen dem Trägerabschnitt 11.1 und dem Tragarm 13 ist ein erstes Drehgelenk 12.1 angeordnet. An einem gegenüberliegenden Ende des Tragarms 13 ist ein zweites Drehgelenk 12.2 angeordnet. Das zweite Drehgelenk 12.2 stellt die Verbindung zum weiteren Tragarm 14 sicher, welcher mit einem dritten Drehgelenk 12.3 verbunden ist, welches die Verbindung zu einer medizintechnischen Einrichtung 20, insbesondere Versorgungskonsole sicherstellt. Wahlweise kann das dritte Drehgelenk 12.3 auch direkt am weiteren Tragarm 14 angeordnet sein. Zwischen der medizintechnischen Einrichtung 20 und dem weiteren Tragarm 14 ist ein Träger 21, insbesondere ein Konsolenrohr angeordnet, welches meist starr ist und eine Drehbewegung zulässt, jedoch nicht notwendigerweise auch einen Höhenausgleich. Ein Höhenausgleich erfolgt bevorzugt durch eine entsprechende Lagerung des weiteren Tragarms 14 am ersten Tragarm 13.

Die medizintechnische Einrichtung 20 weist ein von der Bremseinrichtung entkoppeltes Funktionselement 22 in Form eines Griffs oder einer Stange auf. Ferner weist die medizintechnische Einrichtung 20 ein Tablett 26 in Funktion als "Funktionselement" auf.

Die Deckenstativvorrichtung 1 weist eine Steuerungsvorrichtung 30 auf, die im gezeigten Beispiel an einem der Tragarme angeordnet ist. Wahlweise kann die Steuerungsvorrichtung 30 auch an der medizintechnischen Einrichtung 20 angeordnet sein, wie durch die gestrichelte Linie angedeutet.

Die Steuerungsvorrichtung 30 umfasst am Tragsystem 10 angeordnete Sensoreinrichtungen 31 sowie an der medizintechnischen Einrichtung angeordnete Sensoreinrichtungen 32. Die Sensoreinrichtungen 31 am Tragsystem 10 sind bevorzugt in den Gelenken 12.1, 12.2, 12.3 angeordnet. Die Sensoreinrichtungen 32 an der medizintechnischen Einrichtung 20 sind bevorzugt in Montagepunkten M1, M2 und/oder in Befestigungspunkten B1, B2 angeordnet. Die Montagepunkte M1, M2 und die Befestigungspunkte B1, B2 liefern jeweils eine von außen zugängliche Montage-Schnittstelle für das Funktionselement 22 und das Tablett 26. Eine auf die Deckenstativvorrichtung 1 ausgeübte externe Kraft kann z.B. an den Punkten M1, M2, B1, B2 erfasst werden.

Optional kann Deckenstativvorrichtung 1 die auch eine Anzeigeeinrichtung 40 mit mindestens einem Anzeigeelement (Indikator) 41 aufweisen. Die Anzeigeeinrichtung 40 kann durch die einzelnen Anzeigeelemente 41 gebildet sein und steht in Verbindung mit der Steuerungsvorrichtung 30. Die Steuerungsvorrichtung 30 ist eingerichtet, die Anzeigeeinrichtung 40 als Funktion von erfassten Kraft-Messwerten oder einer absoluten oder relativen Position anzusteuern.

Die Deckenstativvorrichtung 1 weist eine Bremseinrichtung 50 umfassend mindestens eine Bremse 51, 52, 53 auf. Bevorzugt ist an einer jeweiligen Schnittstelle zwischen den Tragarmen 13, 14 und der medizintechnischen Einrichtung 20 jeweils mindestens eine Bremse angeordnet. Die jeweilige Bremse bzw. deren Position ist hier exemplarisch angedeutet. Die Bremseinrichtung 50 kann durch die einzelnen Bremsen 51, 52, 53 gebildet sein und steht in Verbindung mit der Steuerungsvorrichtung 30.

Eine externe Kraft kann mittels der Sensoreinrichtungen 32 direkt in den Montage- und/oder Befestigungspunkten M1, M2, B1, B2 gemessen werden, unabhängig davon, an welchem Punkt des Tabletts 26 oder Funktionselements 22 ein Bediener angreift. Die Sensoreinrichtungen 31, 32 können dabei eingerichtet sein, auch weitere relative oder absolute Größen zu messen, z.B. eine Bewegung oder einen Impuls.

Die Steuerungsvorrichtung 30 ist eingerichtet, mindestens eine der Bremsen 51, 52, 53 in Reaktion auf eine externe Kraft, insbesondere auch in Reaktion auf eine externe Kraft mit einem vorbestimmten Mindest-Betrag oder einer vorbestimmten Richtung oder einem vorbestimmten Richtungsbereich zumindest zu lösen. Hierdurch kann die Deckenstativvorrichtung 1 auf einfache Weise verlagert (z.B. verschwenkt) werden, insbesondere einhändig oder weitgehend "blind".

Die Deckenstativvorrichtung 1 weist ferner eine Antriebseinrichtung 60 umfassend mindestens einen Antrieb 61, 62 auf. Bevorzugt ist an einer jeweiligen Schnittstelle zwischen den Tragarmen 13, 14 und der medizintechnischen Einrichtung 20 jeweils ein Antrieb angeordnet. Der jeweilige Antrieb bzw. dessen Position ist hier exemplarisch angedeutet. Die Antriebseinrichtung 60 kann durch die einzelnen Antriebe 61, 62 gebildet sein und steht in Verbindung mit der Steuerungsvorrichtung 30.

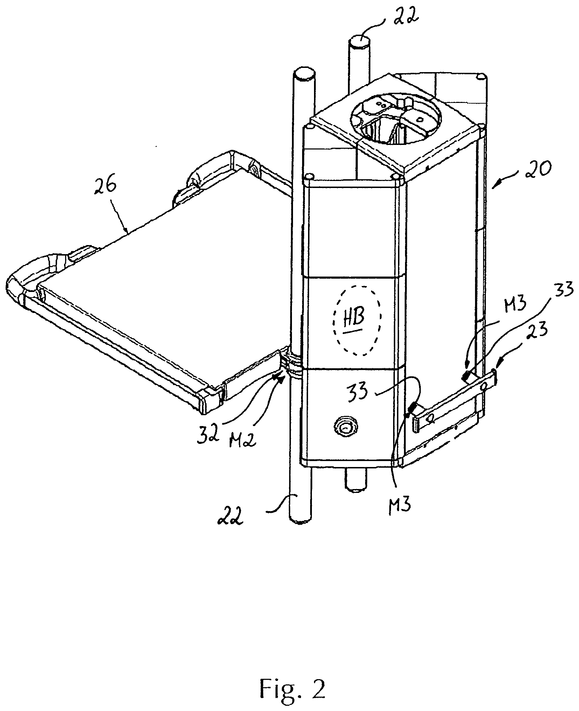

In der Figur 2 ist das Tablett 26 in einer weiteren Perspektive gezeigt. Das Tablett 26 ist in zwei Montagepunkten M2 am Funktionselement 22 befestigt. Die Sensoreinrichtungen 32 können dabei an der Montage-Schnittstelle zwischen dem Funktionselement 22 und dem Tablett 26 und/oder an der Montage-Schnittstelle zwischen dem Funktionselement 22 und dem Gehäuse der medizintechnischen Einrichtung 20 vorgesehen sein.

Ferner ist eine Normschiene 23 gezeigt, welche in weiteren (dritten) Montagepunkten M3 an der Versorgungskonsole befestigt ist, wobei an diesen Montage-Schnittstellen ebenfalls Sensoren 33 (nur schematisch dargestellt) vorgesehen sein können. Zudem ist ein Betätigungsbereich, insbesondere haptischer Betätigungsbereich HB schematisch angedeutet, in welchem eine Bedieneingabe erfolgen kann. Der Betätigungsbereich HB kann dabei auch an anderen ggf. zusätzlichen Stellen der Stativvorrichtung angeordnet sein, insbesondere in Hinblick auf gute Zugänglichkeit je nach Anwendungsfall.

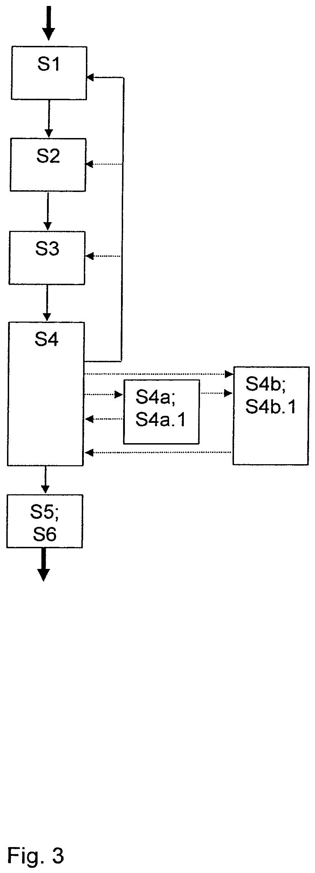

In der Figur 3 sind einzelne Schritte eines Verfahrens zum Positionieren der medizintechnischen Einrichtung gezeigt. Das Verfahren wird durch das Erfassen einer externen Kraft (insbesondere deren Betrag und/oder Richtung) initiiert (Schritt S1). In einem zweiten Schritt S2 erfolgt ein Ausgeben mindestens eines Messwertes der externen Kraft. Der Messwert wird in einem dritten Schritt S3 ausgewertet. In einem vierten Schritt S4 erfolgt ein Ansteuern einer Bremseinrichtung, insbesondere das zumindest graduelle Lösen oder zusätzlich auch wieder ein zumindest graduelles Feststellen mindestens einer Bremse, zum Einstellen eines Bewegungsfreiheitsgrads. Der Schritt S4 kann dabei einen Schritt S4a (Lösen mindestens einer Bremse) umfassen, insbesondere ein graduelles Lösen (Schritt S4a.1). Der Schritt S4 kann dabei auch einen Schritt S4b (Feststellen mindestens einer Bremse) umfassen, insbesondere ein graduelles Feststellen (Schritt S4b.1). Die Schritte S1 bis S4 können iterativ durchgeführt werden, sei es komplett, sei es in Teilschritten. In einem Schritt S5 kann ein Ausgeben eines Signals/Hinweises an einen Bediener erfolgen, insbesondere mittels einer Anzeigeeinrichtung. Ferner kann in einem Schritt S6 ein Ansteuern mindestens eines Antriebs erfolgen. Die Schritte S5 und S6 können parallel zu einem der Schritte S1 bis S4 oder auch nach dem Schritt S4 durchgeführt werden.

### Bezugszeichenliste

- 1: Stativvorrichtung, insbesondere Deckenstativvorrichtung
- 10: Tragsystem
- 11: Montageeinrichtung, insbesondere Deckenflansch oder Bodenabstützung
- 11.1: Trägerabschnitt, insbesondere Säulenstück
- 12.1: (erstes) Drehgelenk
- 12.2: zweites Drehgelenk
- 12.3: drittes Drehgelenk
- 13: (erster) Tragarm
- 14: zweiter Tragarm

- 20: medizintechnische Einrichtung, insbesondere Versorgungskonsole
- 21: Träger, insbesondere Konsolenrohr
- 22: von der Bremseinrichtung entkoppeltes Funktionselement, insbesondere Griff oder Stange
- 23: Funktionselement, insbesondere Normschiene
- 26: Tablett, insbesondere in Funktion als Funktionselement

- 30: Steuerungsvorrichtung
- 31: Sensoreinrichtung am Tragsystem
- 32: Sensoreinrichtung an der medizintechnischen Einrichtung
- 33: Sensoreinrichtung, insbesondere an/in Montage-Schnittstelle

- 40: Anzeigeeinrichtung
- 41: Anzeigeelement (Indikator)

- 50: Bremseinrichtung
- 51: (erste) Bremse
- 52: zweite Bremse
- 53: dritte Bremse

- 60: Antriebseinrichtung
- 61: (erster) Antrieb, insbesondere Drehantrieb
- 62: zweiter Antrieb, insbesondere Drehantrieb
- B1: (erster) Befestigungspunkt
- B2: zweiter Befestigungspunkt

- HB: Betätigungsbereich, insbesondere haptischer Betätigungsbereich

- M1: (erster) Montagepunkt
- M2: zweiter Montagepunkt
- M3: dritter Montagepunkt

- S1: erster Schritt: Erfassen einer externen Kraft
- S2: zweiter Schritt: Ausgeben eines Messwertes
- S3: dritter Schritt: Auswerten des Messwertes
- S4: vierter Schritt: Ansteuern einer Bremseinrichtung, insbesondere zumindest graduelles Lösen einer Bremse, zum Einstellen eines Bewegungsfreiheitsgrads
- S4a: Lösen einer Bremse
- S4a.1: graduelles Lösen
- S4b: Feststellen einer Bremse
- S4b.1: graduelles Feststellen
- S5: Ausgeben eines Signals/Hinweises an einen Bediener
- S6: Ansteuern mindestens eines Antriebs

## Patentansprüche

1. Stativvorrichtung (1) zur Anordnung im Operationssaal und zum örtlichen Verlagern einer an der Stativvorrichtung (1) montierten medizintechnischen Einrichtung (20), umfassend
- mindestens einen bewegbar gelagerten Tragarm (13,14);
- ein Tragsystem (10) umfassend eine Abstützeinrichtung (11);
- eine Bremseinrichtung (50) mit mindestens einer Bremse (51), die eingerichtet ist, einen Bewegungsfreiheitsgrad des mindestens einen Tragarms (13, 14) und/oder der medizintechnischen Einrichtung (20) relativ zur Abstützeinrichtung (11) einzustellen;
wobei die Stativvorrichtung (1) ferner umfasst:
eine mit der Bremseinrichtung (50) in Verbindung stehende Steuerungseinrichtung (30) und
mindestens eine mit der Steuerungseinrichtung (30) in Verbindung stehende Sensoreinrichtung (31, 32), eingerichtet zum Erfassen einer auf die Stativvorrichtung (1) einwirkenden externen Kraft und/oder einer durch die externe Kraft hervorgerufenen Bewegung und eingerichtet zum Ausgeben eines entsprechenden Messwerts an die Steuerungseinrichtung (30),
wobei die Steuerungseinrichtung (30) eingerichtet ist, die Bremseinrichtung (50) in Abhängigkeit des Messwerts anzusteuern und den Bewegungsfreiheitsgrad einzustellen, und
wobei die Steuerungseinrichtung (30) eingerichtet ist, in Abhängigkeit von einer zeitlichen Änderung, einem Betrag und/oder einer Richtung der externen Kraft und/oder einer Richtung und/oder einer Geschwindigkeit der Bewegung den Bewegungsfreiheitsgrad der Stativvorrichtung (1) einzustellen,
**dadurch gekennzeichnet, dass**
die Steuerungseinrichtung (30) eingerichtet ist, die Bremseinrichtung (50) in Abhängigkeit des Messwerts autonom und ohne Betätigung eines zusätzlichen Schalters ausschließlich aufgrund der auf die Stativvorrichtung (1) einwirkenden externen Kraft zu lösen.

2. Stativvorrichtung (1) nach Anspruch 1, **dadurch gekennzeichnet, dass** die Steuerungseinrichtung (30) eine Uhr, einen Timer oder einen Zeitgeber aufweist.

3. Stativvorrichtung (1) nach Anspruch 1 oder 2, **dadurch gekennzeichnet, dass** die Steuerungseinrichtung (30) eingerichtet ist, die Bremseinrichtung (50) in Abhängigkeit eines Kraft- oder Momentenanstiegs zu lösen.

4. Stativvorrichtung (1) nach einem der vorhergehenden Ansprüche, **dadurch gekennzeichnet, dass** die Bremseinrichtung (50) mehrere Bremsen umfasst, welche jeweils an einem jeweiligen Tragarm und/oder an der medizintechnischen Einrichtung (20) angeordnet sind, wobei die Steuerungseinrichtung (30) eingerichtet ist, die Bremseinrichtung (50) selektiv in Bezug auf den mindestens einen Tragarm und/oder in Bezug auf die medizintechnische Einrichtung (20) in Abhängigkeit der Richtung der externen Kraft oder Bewegung einzustellen.

5. Stativvorrichtung (1) nach einem der vorhergehenden Ansprüche, **dadurch gekennzeichnet, dass** die Steuerungseinrichtung (30) eingerichtet ist, die mindestens eine Bremse (51) in Abhängigkeit des Betrags oder der Richtung der externen Kraft und/oder der Richtung der Bewegung und/oder des Weges der Bewegung zumindest graduell zu lösen und/oder zumindest graduell festzustellen, oder dass die Steuerungseinrichtung (30) eingerichtet ist, die mindestens eine Bremse (51) in Abhängigkeit eines von einer Positionssensoreinrichtung erfassten Positionssignals einzustellen, bevorzugt bei Stillstand der Stativvorrichtung (1), bevorzugt bei Stillstand nach einer vordefinierten Mindestdauer.

6. Stativvorrichtung (1) nach einem der vorhergehenden Ansprüche, **dadurch gekennzeichnet, dass** die Stativvorrichtung (1) mindestens ein manuell betätigbares Funktionselement (22, 23, 26) aufweist, beispielsweise ein Tablett, an welchem eine Sensoreinrichtung der mindestens einen Sensoreinrichtung angeordnet ist, bevorzugt mindestens ein Kraftsensor.

7. Stativvorrichtung (1) nach einem der vorhergehenden Ansprüche, **dadurch gekennzeichnet, dass** die Stativvorrichtung (1) die medizintechnische Einrichtung (20) umfasst und eine Montage-Schnittstelle aufweist, in welcher die externe Kraft erfassbar ist, wobei die Montage-Schnittstelle an der medizintechnischen Einrichtung (20) vorgesehen ist, und wobei in der Montage-Schnittstelle ein erstes Funktionselement (22, 23, 26), beispielsweise in Form eines Griffs oder einer Stange (22), befestigt ist.

8. Stativvorrichtung (1) nach einem der vorhergehenden Ansprüche, **dadurch gekennzeichnet, dass** die Stativvorrichtung (1) einen Betätigungsbereich aufweist, welcher von außen zugänglich ist und an welchem die externe Kraft in die Stativvorrichtung (1) einleitbar ist.

9. Stativvorrichtung (1) nach einem der vorhergehenden Ansprüche, **dadurch gekennzeichnet, dass** die Stativvorrichtung (1) mindestens einen in Verbindung mit der Steuerungseinrichtung stehenden Antrieb zum motorischen Bewegen mindestens eines Tragarms aufweist, wobei die Steuerungseinrichtung (30) eingerichtet ist, die durch eine externe Kraft hervorgerufene Bewegung in Abhängigkeit eines Betriebszustands des mindestens einen Antriebs auszuwerten.

10. Stativvorrichtung (1) nach einem der vorhergehenden Ansprüche, **dadurch gekennzeichnet, dass** wenigstens eine Sensoreinrichtung der mindestens einen Sensoreinrichtung (31, 32) in einem Drehgelenk der Stativvorrichtung (1) angeordnet ist.

11. Stativvorrichtung (1) nach einem der vorhergehenden Ansprüche, **dadurch gekennzeichnet, dass** die mindestens eine Sensoreinrichtung (31, 32) ein Kraftsensor aus der Gruppe Drucksensor, Dehnungsmessstreifen und Torsionssensor ist.

12. Verfahren zum Positionieren einer medizintechnischen Einrichtung (20) an einer im Operationssaal anordenbaren Stativvorrichtung (1) gemäß Anspruch 1, **gekennzeichnet durch** die Schritte:
- Erfassen einer auf die Stativvorrichtung (1) einwirkenden externen Kraft oder einer durch die externe Kraft hervorgerufenen Bewegung;
- Ausgeben eines entsprechenden Messwerts an eine Steuerungseinrichtung (30) der Stativvorrichtung (1);
- Auswerten des Messwerts mittels der Steuerungseinrichtung (30); und
- Ansteuern einer Bremseinrichtung (50) der Stativvorrichtung (1) und Einstellen eines Bewegungsfreiheitsgrads der medizintechnischen Einrichtung (20) bzw. der Stativvorrichtung (1) mittels der Steuerungseinrichtung (30) in Abhängigkeit von dem Messwert betreffend eine zeitliche Änderung, einen Betrag und/oder eine Richtung der externen Kraft, und/oder eine Richtung und/oder eine Geschwindigkeit der Bewegung,
wobei die Bremseinrichtung (50) mittels der Steuerungseinrichtung (30) in Abhängigkeit des Messwerts autonom und ohne Betätigung eines zusätzlichen Schalters ausschließlich aufgrund der auf die Stativvorrichtung (1) einwirkenden externen Kraft gelöst wird.

13. Verfahren nach Anspruch 12, **dadurch gekennzeichnet, dass** das Ansteuern der Bremseinrichtung (50) ein zumindest graduelles Lösen mindestens einer Bremse der Bremseinrichtung umfasst, sobald eine externe Kraft erfasst wird oder wenn die externe Kraft erstmals einen bestimmten Schwellwert überschreitet oder in einer bestimmten Richtung ausgeübt wird, und/oder dass die Bremseinrichtung (50) graduell fester gestellt wird oder festgestellt wird, sobald sich die externe Kraft vermindert oder keine externe Kraft mehr erfasst wird, oder sobald sich die Richtung der externen Kraft ändert, bevorzugt ab einer Änderung der Richtung in Abhängigkeit von einem Mindestschwellwert.

14. Verfahren nach einem der Ansprüche 12 oder 13, **dadurch gekennzeichnet, dass** die durch eine externe Kraft hervorgerufene Bewegung in Abhängigkeit eines Betriebszustands mindestens eines Antriebs der Stativvorrichtung (1) ausgewertet wird.

## Claims

1. Stand device (1) for arranging in an operating room and for locally moving a medical device (20) mounted on the stand device (1), comprising
- at least one movably mounted support arm (13, 14);
- a support system (10) comprising a mounting device (11;
- a braking device (50) having at least one brake (51) that is configured to adjust a degree of freedom of movement of the at least one support arm (13, 14) and/or the medical device (20) relative to the supporting device (11);
wherein the stand device (1) further comprises:
a control device (30) connected to the braking device (50) and
at least one sensor device (31, 32) connected to the control device (30), the sensor device (30) being configured to detect an external force acting on the stand device (1) and/or a movement caused by the external force and configured to output a corresponding measured value to the control device (30),
wherein the control device (30) is configured to control the braking device (50) as a function of the measured value and adjust the degree of freedom of movement, and
wherein the control device (30) is configured to adjust the degree of freedom of movement of the stand device (1) as a function of a temporal change, a magnitude and/or a direction of the external force and/or a direction and/or a velocity of the movement,
**characterized in that**
the control device (30) is configured to release a braking device (50) autonomously and without actuation of an additional switch exclusively by the external force acting on the stand device (1) by virtue of the control device (30) as a function of the measured value.

2. Stand device (1) according to claim 1, **characterized in that** the control device (30) is a clock, a timer or a time-switch.

3. Stand device (1) according to claim 1 or 2, **characterized in that** the control device (30) is configured to release the braking device (50) as a function of the force or torque increase.

4. Stand device (1) according to any of the preceding claims **characterized in that** the braking device (50) comprises several brakes that are each arranged on a respective support arm and/or on the medical device (20), wherein the control device (30) is configured to selectively adjust the breaking device (50) with respect to the at least one support arm and/or with respect to the medical device (20) as a function of the direction of the external force or movement.

5. Stand device (1) according to any of the preceding claims, **characterized in that** the control device (30) is configured to at least gradually release and/or at least gradually apply the at least one brake (51) as a function of the magnitude or the direction of the external force and/or the direction of the movement and/or the path of the movement, or **in that** the control device (30) is configured to adjust the at least one brake (51) as a function of a position signal detected by a position sensor device, preferably when the stand device (1) is at a standstill, and preferably at a standstill after a predefined minimum duration.

6. Stand device (1) according to any of the preceding claims, **characterized in that** the stand device (1) has at least one manually operable functional element (22, 23, 26), particularly a tray on which a sensor device of the at least one sensor device is arranged, preferably, at least one force sensor.

7. Stand device (1) according to any of the preceding claims, **characterized in that** the stand device (1) comprises the medical device (20) and has a mounting interface in which the external force can be detected, wherein the mounting interface is provided on the medical device (20), and wherein a first functional element (22, 23, 26) - e. g. in the form of a handle or a bar (22) - is fixed in the mounting interface.

8. Stand device (1) according to any of the preceding claims, **characterized in that** the stand device (1) has an operating area that is accessible from the outside and in which the external force can be introduced into the stand device (1).

9. Stand device (1) according to any of the preceding claims, **characterized in that** the stand device (1) has at least one drive connected to the control device for the motorized movement of at least one support arm, wherein the control device (30) is configured to evaluate the movement caused by an external force as a function of an operating state of the at least one drive.

10. Stand device (1) according to any of the preceding claims, **characterized in that** at least one sensor device of the at least one sensor device (31, 32) is arranged in a swivel joint of the stand device (1).

11. Stand device (1) according to any of the preceding claims, **characterized in that** the at least one sensor device (31, 32) is a force sensor from the group including pressure sensors, strain gauges, and torsion sensors.

12. Method for positioning a medical device (20) on a stand device (1) that can be arranged in an operating room according to claim 1, **characterized by** the steps:
- detecting an external force acting on the stand device (1) or a movement caused by the external force;
- outputting a corresponding measured value to a control device (30) of the stand device (1);
- evaluating the measured value by means of the control device (30); and
- controlling a braking device (50) of the stand device (1) and adjusting the degree of freedom of movement of the medical device (20) or of the stand device (1), respectively, by means of the control device (30) as a function of the measured value referring to a temporal change, a magnitude and/or a direction of the external force, and/or a direction and/or a velocity of the movement,
wherein the braking device (50) is released autonomously and without actuation of an additional switch exclusively by the external force acting on the stand device (1) by virtue of the control device (30) as a function of the measured value.

13. Method according to claim 12, **characterized in that** controlling the braking device (50) comprises an at least gradual release of at least one brake of the braking device as soon as an external force is detected or if the external force exceeds a certain threshold value for the first time or is exerted in a specific direction, and/or **in that** the braking device (50) is fully or gradually applied, as soon as the external force decreases or no external force is detected anymore, or as soon as the direction of the external force changes, preferably starting from a change in direction as a function of a minimum threshold value.

14. Method according to one of claims 12 or 13, **characterized in that** the movement caused by an external force is evaluated as a function of an operating state of at least one drive of the stand device (1).

## Revendications

1. Dispositif à trépied (1) destiné à être agencé dans une salle d'opération et destiné au déplacement local d'un équipement médical (20) monté au niveau du dispositif à trépied (1), comprenant
- au moins un bras porteur logé de manière mobile (13, 14) ;
- un système porteur comprenant un équipement de support (11) ;
- un équipement de freinage (50) avec au moins un frein (51) qui est mis en place pour régler un degré de liberté de mouvement de l'au moins un bras porteur (13, 14) et/ou de l'équipement médical (20) par rapport à l'équipement de support (11) ;
dans lequel le dispositif à trépied (1) comprend en outre :
un équipement de commande (30) en liaison avec l'équipement de freinage (50) et
au moins un équipement de détection (31, 32) en liaison avec l'équipement de commande (30), mis en place pour l'acquisition d'une force externe agissant sur le dispositif à trépied (1) ou d'un mouvement provoqué par le biais de la force externe et mis en place pour la délivrance d'une valeur de mesure correspondante à l'équipement de commande (30),
dans lequel l'équipement de commande (30) est mis en place pour commander l'équipement de freinage (50) en fonction de la valeur de mesure et régler le degré de liberté de mouvement, et
dans lequel l'équipement de commande (30) est mis en place pour régler le degré de liberté de mouvement du dispositif à trépied (1) en fonction d'une modification temporelle, d'une ampleur et/ou d'une direction de la force externe et/ou d'une direction et/ou d'une vitesse du mouvement,
**caractérisé en ce que** l'équipement de commande (30) est mis en place pour déclencher l'équipement de freinage (50) en fonction de la valeur de mesure de manière autonome et sans actionnement d'un commutateur supplémentaire exclusivement en raison de la force externe agissant sur le dispositif à trépied (1).

2. Dispositif à trépied (1) selon la revendication 1, **caractérisé en ce que** l'équipement de commande (30) présente une montre, un minuteur ou un temporisateur.

3. Dispositif à trépied (1) selon la revendication 1 ou 2, **caractérisé en ce que** l'équipement de commande (30) est mis en place pour déclencher l'équipement de freinage (50) en fonction d'une augmentation de force ou d'une montée en couple.

4. Dispositif à trépied (1) selon l'une quelconque des revendications précédentes, **caractérisé en ce que** l'équipement de freinage (50) comprend plusieurs freins, qui sont agencés respectivement au niveau d'un bras porteur respectif et/ou au niveau de l'équipement médical (20), dans lequel l'équipement de commande (30) est mis en place pour régler l'équipement de freinage (50) sélectivement par rapport à l'au moins un bras porteur et/ou par rapport à l'équipement médical (20) en fonction de la direction de la force externe ou du mouvement.

5. Dispositif à trépied (1) selon l'une quelconque des revendications précédentes, **caractérisé en ce que** l'équipement de commande (30) est mis en place pour déclencher au moins graduellement et/ou pour enclencher au moins graduellement l'au moins un frein (51) en fonction de l'ampleur ou de la direction de la force externe et/ou de la direction du mouvement et/ou de la course du mouvement ou **en ce que** l'équipement de commande (30) est mis en place pour régler l'au moins un frein (51) en fonction d'un signal de position acquis par un équipement de détection de position, de préférence lorsque le dispositif à trépied (1) est à l'arrêt, de préférence lorsqu'il est à l'arrêt après une durée minimale prédéfinie.

6. Dispositif à trépied (1) selon l'une quelconque des revendications précédentes, **caractérisé en ce que** le dispositif à trépied (1) présente au moins un élément fonctionnel (22, 23, 26) actionnable manuellement, par exemple une tablette, au niveau de laquelle un équipement de détection de l'au moins un équipement de détection est agencé, de préférence au moins un détecteur de force.

7. Dispositif à trépied (1) selon l'une quelconque des revendications précédentes, **caractérisé en ce que** le dispositif à trépied (1) comprend l'équipement médical (20) et présente une interface de montage, dans laquelle la force externe est **détectable,** dans lequel l'interface de montage est prévue au niveau de l'équipement médical (20), et dans lequel dans l'interface de montage un premier élément fonctionnel (22, 23, 26), par exemple en forme de poignée ou de perche (22), est fixé.

8. Dispositif à trépied (1) selon l'une quelconque des revendications précédentes, **caractérisé en ce que** le dispositif à trépied (1) présente une zone d'activation qui est accessible par l'extérieur et au niveau de laquelle la force externe peut être introduite dans le dispositif à trépied (1).

9. Dispositif à trépied (1) selon l'une quelconque des revendications précédentes, **caractérisé en ce que** le dispositif à trépied (1) présente au moins un entraînement en liaison avec l'équipement de commande et destiné au déplacement motorisé d'au moins un bras porteur, dans lequel l'équipement de commande (30) est mis en place pour évaluer le mouvement provoqué par le biais de la force externe en fonction d'un état de fonctionnement de l'au moins un entraînement.

10. Dispositif à trépied (1) selon l'une quelconque des revendications précédentes, **caractérisé en ce qu'**au moins un équipement de détection de l'au moins un équipement de détection (31, 32) est agencé dans une articulation de rotation du dispositif à trépied (1).

11. Dispositif à trépied (1) selon l'une quelconque des revendications précédentes, **caractérisé en ce que** l'au moins un équipement de détection (31, 32) est un détecteur de force issu du groupe composé d'un détecteur de pression, d'une jauge de contrainte et d'un détecteur de torsion.

12. Procédé destiné au positionnement d'un équipement médical (20) au niveau d'un dispositif à trépied (1) pouvant être agencé dans une salle d'opération selon la revendication 1, **caractérisé par** les étapes suivantes :
- acquisition d'une force externe agissant sur le dispositif à trépied (1) ou d'un mouvement provoqué par le biais de la force externe ;
- délivrance d'une valeur de mesure correspondante à un équipement de commande (30) du dispositif à trépied (1) ;
- évaluation de la valeur de mesure au moyen de l'équipement de commande (30) ; et
- commande d'un équipement de freinage (50) du dispositif à trépied (1) et réglage d'un degré de liberté de mouvement de l'équipement médical (20) resp. du dispositif à trépied (1) au moyen de l'équipement de commande (30) en fonction de la valeur de mesure concernant une modification temporelle, une ampleur et/ou une direction de la force externe et/ou une direction et/ou une vitesse du mouvement,
dans lequel l'équipement de freinage (50) est déclenché au moyen de l'équipement de commande (30) en fonction de la valeur de mesure de manière autonome et sans activation d'un commutateur supplémentaire exclusivement en raison de la force externe agissant sur le dispositif à trépied (1).

13. Procédé selon la revendication 12, **caractérisé en ce que** la commande de l'équipement de freinage (50) comprend un déclenchement au moins graduel d'au moins un frein de l'équipement de freinage dès qu'une force externe est acquise ou lorsque la force externe dépasse pour la première fois un seuil déterminé ou est exercée dans une direction déterminée et/ou **en ce que** l'équipement de freinage (50) est placé graduellement plus fermement ou est enclenché dès que la force externe diminue ou qu'aucune force externe n'est plus acquise ou dès que la direction de la force externe est modifiée, de préférence à partir d'une modification de la direction en fonction d'une valeur de seuil minimale.

14. Procédé selon l'une quelconque des revendications 12 ou 13, **caractérisé en ce que** le mouvement provoqué par le biais d'une force externe est évalué en fonction d'un état de fonctionnement au moins d'un entraînement du dispositif à trépied (1).
